# EUROPEAN PATENT SPECIFICATION

(11) **EP 3 854 209 B1**
(45) Date of publication and mention of the grant of the patent: **13.11.2024**
(21) Application number: 19862136.9
(22) Date of filing: 23.07.2019
(51) Int. Cl.: A01K 67/033, A23K 50/90, A61K 35/64, A61P 17/18, A61P 29/00, A61P 17/12

(54) **METHOD OF PRODUCING BIOMASS OF GALLERIA MELLONELLA LARVAE**
VERFAHREN ZUR HERSTELLUNG VON BIOMASSE VON LARVEN VON GALLERIA MELLONELLA
PROCÉDÉ DE PRODUCTION DE BIOMASSE DE LARVES DE GALLERIA MELLONELLA

(30) Priority: 19.09.2018 MD 20180081
(43) Date of publication of application: 28.07.2021
(73) Proprietor: Ciuhrii, Ceslav, 2021 Chisinau (MD)
(72) Inventor: Ciuhrii, Ceslav, 2021 Chisinau (MD)
(74) Representative: Puscasu, Dan
(86) International application number: PCT/MD2019/000005
(87) International publication number: WO 2020/060382

(56) References cited:
- CN-A- 107 182 948
- CN-A- 107 182 948
- MD-C1- 4 198
- RU-C1- 2 038 086
- RU-C1- 2 038 086
- ADELINE L. JORJAO ET AL: "From moths to caterpillars: Ideal conditions for Galleria mellonella rearing for in vivo microbiological studies", VIRULENCE, vol. 9, no. 1, March 2018 (2018-03-01), US, pages 383 - 389, XP055693529, ISSN: 2150-5594, DOI: 10.1080/21505594.2017.1397871
- BRASLAVSKY, V. B: "Issledovanie elektronnykh spektrov flovonoidov topolia i propoliusa = [Study of the electronic spectra of poplar and propolius flavonoids]", MEDITSINSKY ALMANAKH, vol. 2, no. 15, 2011, pages 140 - 144, XP009527168, ISSN: 1997-7689
- ADELINE L JORJÃO; LUCIANE D OLIVEIRA; LILIANA SCORZONI; LÍVIA MARA A FIGUEIREDO-GODOI; MARCIA CRISTINA A PRATA; ANTONIO OLAVO C J: "From moths to caterpillars: ideal conditions for Galleria mellonella rearing for in vivo microbiological studies", VIRULENCE, vol. 9, no. 1, 31 December 2018 (2018-12-31), pages 383 - 389, XP055693529, ISSN: 2150-5594, DOI: 10.1080/21505594.2017.1397871

## Description

The invention relates to biotechnology, in particular to the obtainment of the entomological biomass - *Galleria mellonella* larvae with high biological activity (for example, keratolytic, antioxidant, anti-inflammatory, immunomodulating, etc.), which can be used as raw materials for the production of the pharmaceutical preparations and cosmetic products.

It is known the method of the obtainment of the biomass of *Galleria mellonella* larvae, which includes the cultivation of larvae of the first-second age on the nutrient medium that contains the wheat flour (110 g), bakery yeast (100 g), milk powder (70 g), wax (50 g), glycerine (80 g), wheat bran (350 g), bee honey (50 g) and water (160 g); and larvae of the third-fourth age - on the medium that contains the wheat bran (350 g), wheat flour (110 g), bakery yeast (100 g), milk powder (70 g), wax (50 g), glycerine (80 g), water (210 g) [1]. Larvae are intended for use as an organism - the host for various pathogens in the scientific researches [1].

The disadvantage of this method is the use of poor nutrient medium for the cultivation of the larvae of the third-fourth age, which cannot provide a high biological value of the biomass.

It is also known the method of the obtainment of the biomass of *Galleria mellonella* larvae, including the incubation of the larvae of the last age or the pupae in the containers, in the darkness for 1-3 weeks for the obtainment of the maximum number of eggs, transfer of eggs (twice a week) to the food containers for 20 days for the obtainment of larvae. The nutrient medium used for the feeding of larvae has the following composition: corn flour 250 g, yeast extract 150 g, soy flour 100 g, milk powder 100 g, bee honey 200 g, glycerine 200 g, blocks of beeswax. Larvae are characterized by high resistance to the infections of *Staphylococcus aureus, Escherichia coli* and *Candida albicans* and are used as the hosts in the study of entomopathogens [2].

The disadvantage of the method is that the obtained larvae vary significantly in dimensions (from 1 to 2 cm) and quality of biomass because of different ages that is unacceptable, when the biomass is used as the raw material for the production of the cosmetic or pharmaceutical substances.

It is known also the method of the obtainment of *Galleria mellonella* larvae that consists in the fact that the eggs of the insects are placed in the plastic boxes with the nutrient medium, where they are developed in the course of 51 days. The used nutrient medium has the following composition: 118 g of wheat flour, 206 g of wheat bran, 118 g of milk powder, 88 g of beer yeast, 24 g of wax powder, 175 ml of honey and 175 ml of glycerin. The obtained larvae are used as the host organism for the cultivation of the entomophagous nematodes [3].

The disadvantage of this method consists in the 51-day duration of the process of growth that leads to a significant increase in the cost of the technology of cultivation of insects.

The closest to the claimed invention is the method of the obtainment of the biomass of *Galleria mellonella* larvae [4] for the use as the raw materials for the production of cosmetic and pharmaceutical preparations having the keratolytic effect. This process includes three consecutive stages: **1. Preparation of conditions for the passage of the full life cycle under the conditions of artificial cultivation.** For the cultivation of the insects are used the premises with the area of 20 m². The walls are painted with a special resin that makes it possible the disinfection by the disinfectant solutions. In the premises where the eggs are incubated, the temperature is maintained at the level of 26 ± 2 °C, and humidity - 70%. The premises in which the larvae are cultivated are equipped with the dark boxes and provided with the automatic temperature and humidity control systems. In these premises it is maintained the temperature of 30-31 °C and humidity of 80%; **2. Cultivation of insects.** Immediately after the hatching, the larvae are transferred from the incubation rooms to the premises intended for the cultivation of larvae. Larvae are placed in the dark containers, where the necessary conditions are maintained, and the insects receive the necessary nutrition - the nutrient medium of the following composition: yellow corn extract 174,0-206,0 g, red corn extract 183,0-214,0 g, glucose 70,0-85,0 g, fructose 65,0- 81,0 g, sucrose 4,0-4,8 g, maltose 4,0-5,0 g, casein 117,0-148,0, retinol (vitamin A) 2500-3000 un., tocopherol acetate (vitamin E) 40-58 un., vitamin C 0,05-0,06 g, riboflavin (vitamin B₂) 0,0014-0,0017 g, calcium pantothenate (vitamin B₅) 0,005-0,01, thiamine (Vitamin B₁) 0,002-0,003 g, pyridoxine 0,003-0,004 g, biotin 0,00002-0,00003 g, niacin (vitamin B₃) 0,0002-0,0003 g, pinobanksin 0,015-0,020 g, pinocembrin 0,010-0,015 g, glycerin 120,0-139,0 g, NaNO₃ 0,8-1,1 g, KH₂PO₄ 1,7-2,0 g, NaCl 1,5-2,0 g, CaCl₂ 0,2-0,5, MgSO₄ - 7H₂O 0,05-0,1 g, ZnSO₄ * 7H₂O 0,005-0,01 g, MnSO₄ * 5H₂O 0,007-0,015 g, CuSO₄ * 5 H₂O 0,007 0,008 g, FeCl₃ * 6H₂O 0,0100-0,0175 g, EDTA 0,006-0,0075 g, yellow wax 250-350 g, purified water - up to 1000 g. **3. Collection of larvae.** Larvae are collected at the entrance in the 7^{th} age, immediately after the moulting. This biomass is used for the obtainment of the active complex having the keratolytic effect.

The disadvantage of this method is the use of a very complex nutrient medium, consisting of more than 30 components, as well as the collection of larvae immediately after moulting, when the amount of the ferment having the keratolytic effect decreases as a result of their use in the process of the removal of the old exoskeleton.

Also, the disadvantage of all methods described above is that the development of the larval stage of the insect *Galleria mellonella* that includes from 3 to 10 moults (depending on the given example), occurs in the same containers in which were placed the eggs or the larvae of the first-second age. This leads to the accumulation of the waste products of insects and the decrease of the yield of biomass.

The problem solved by the given invention consists in the fact of the elaboration of a new effective method for the obtainment of the biomass of *Galleria mellonella* larvae with high biological activity.

The essence of the invention consists in the fact that it is proposed the method of the obtainment of the biomass of *Galleria mellonella* larvae with high biological activity that consists in the preparation of the conditions for the cultivation of larvae under the artificial conditions, cultivation of larvae on a nutrient medium and collection of larvae, and the nutrient medium for *Galleria mellonella* larvae has the following composition, g/kg:

| | |
|---|---|
| Wheat flour | 185-200 g |
| Corn flour | 180-190 g |
| Wheat bran | 110-130 g |
| Yeast extract | 15-20 g |
| Powdered milk | 120-140 g |
| Bee honey | 75-95 g |
| Glycerine, | 80-90 g |
| Wax powder | 40-55 g |
| Pinobanksin | 0,02-0,03 g |
| Pinocembrin | 0,02-0,03 g |
| Purified water up to 1000 g, | |

and during the period of the cultivation of larvae, at least 2 transfers to fresh nutrient medium are carried out, the larvae are collected at different ages depending on the type of the required biological activity.

The technical result of the invention, in comparison with the closest solution, consists in the fact that the method of the obtainment of the biomass of *Galleria mellonella* larvae includes a simpler and less expensive medium that provides a larger amount of biomass (149,7 - 160,0 mg of the biomass on average for 1 g compared with 113,6 - 121,3 for the prototype) with a higher biological activity.

The technical result of the invention is due to the fact that during the development of *Galleria mellonella* larvae, at least two transfers of larvae to a fresh medium are carried out that prevents the effect of inhibition caused by the accumulation of the waste products in the containers for the cultivation of insects.

### Examples of application of the invention:

### Example 1:

### Obtainment of the biomass of Galleria mellonella larvae with keratolytic activity

Pupae of *Galleria mellonella* are taken out the refrigerator, where were stored at the temperature of 3-5 °C and are left at the indoor temperature for 2 hours. With the passage of this time, the pupae are transferred to the closed containers containing a small amount of nutrient medium (1/10 of the volume of the container) for their subsequent development and transition to the phase of imago and oviposition. For the initiation of the hatching of larvae from eggs in the dark containers, it is maintained the temperature of 27 °C and the relative humidity of 75%. The first instar larvae appear 48 hours after the pupae are placed in the containers. The process is monitored, over a period of time during which 80% yield is provided, but not more than 6 hours.

The used nutrient medium has the following composition, g/kg:

| | |
|---|---|
| Wheat flour | 185 g |
| Corn flour | 180 g |
| Wheat bran | 110 g |
| Yeast extract | 15 g |
| Powdered milk | 120 g |
| Bee honey | 75 g |
| Glycerine, | 80 g |
| Wax powder | 40 g |
| Pinobanksin | 0,02 g |
| Pinocembrin | 0,02 g |
| Purified water up to 1000 g, | |

New containers with nutrient medium are prepared. 6 hours after the start of the hatching, the larvae of the first generation are selected by the tweezers from the containers in which they appeared and are placed in the prepared containers.

Growth of larvae lasts 20 days since the date of the first transfer. The temperature of the container is maintained at the level of 27 °C and the relative humidity constitutes 75% in the dark conditions.

During the given period, on the 7^{th} day and the 14^{th} day, two transfers of larvae to a fresh nutrient medium are carried out.

For the obtainment of the biomass of larvae with a high keratolytic activity, the collection of larvae occurs on the 19^{th} day of cultivation, before the beginning of the 6^{th} moulting of larvae. Larvae are washed twice by four times volumes of physiological solution and twice more by four times volumes of purified water.

In the obtained biomass are determined the quantitative and qualitative indicators. The obtained results are demonstrated in the table 1.

**Table 1**

| **Parameter** | **According to the closest solution** | **According to the proposed method** |
|---|---|---|
| Cost of one kg of nutrient medium | 100% | 25% |
| Amount of biomass of larvae (mg) obtained from one g of medium | 113,6±6,2 | 149,7±2,9 |
| Keratolytic activity, U/mg of dry matter | 1,96±0,05 | 4,05±0,04 |

The data in the Table 1 prove that the use of a simplified medium leads to a significant reduction of the cost of the production of larvae (the cost of food in the proposed solution constitutes 25% of the cost of the prototype medium), and the amount of the biomass produced using one gram of medium is 31,8% more compared to the prototype. At the same time the keratolytic activity of the biomass is doubled.

### Example 2:

### Obtainment of the biomass of Galleria mellonella larvae with high antioxidant activity

Pupae of *Galleria mellonella* are taken out the refrigerator, where were stored at the temperature of 3-5 °C and are left at the indoor temperature for 2 hours. With the passage of this time, the pupae are transferred to the closed containers containing a small amount of the nutrient medium (1/10 of the volume of the container) for their subsequent development and transition to the phase of imago and oviposition. For the initiation of the hatching of larvae from eggs in the dark containers, it is maintained the temperature of 27 °C and a relative humidity of 75%. The first instar larvae appear 48 hours after the pupae are placed in the containers. The process is monitored, over a period of time during which 80% yield is provided, but not more than 6 hours.

The used nutrient medium has the following composition, g/kg:

| | |
|---|---|
| Wheat flour | 200 g |
| Corn flour | 190 g |
| Wheat bran | 130 g |
| Yeast extract | 20 g |
| Powdered milk | 140 g |
| Bee honey | 95 g |
| Glycerine, | 90 g |
| Wax powder | 55 g |
| Pinobanksin | 0,03 g |
| Pinocembrin | 0,03 g |
| Purified water up to 1000 g; | |

New containers with nutrient medium are prepared. 6 hours after the start of the hatching, the larvae of the first generation are selected by the tweezers from the containers in which they appeared and are placed in the prepared containers.

Growth of larvae lasts 20 days since the date of the first transfer. The temperature of the container is maintained at the level of 27 °C and the relative humidity constitutes 75% in the dark conditions.

During the given period, on the 7^{th} day and the 14^{th} day, two transfers of larvae to a fresh nutrient medium are carried out.

For the obtainment of the biomass of larvae with a high antioxidant activity, the collection of larvae occurs on the 20^{th} day of cultivation. Larvae are washed twice by four times volumes of physiological solution and twice more by four times volumes of purified water.

In the obtained biomass are determined the quantitative and qualitative indicators. The obtained results are demonstrated in the table 2.

**Table 2**

| **Parameter** | **According to the closest solution** | **According to the proposed method** |
|---|---|---|
| Cost of one kg of nutrient medium | 100% | 25% |
| Amount of biomass of larvae (mg) obtained from one g of medium | 118,4 | 156,1 |
| Antioxidant activity, Trolox mg-equivalent / g of the biomass | 28,4±0,2 | 36,8±0,6 |

The data in the Table 2 demonstrate that the amount of the biomass obtained using one gram of medium is increased by 31,7% compared with the prototype. Also, it is increased the antioxidant activity of the biomass by 1,3 times.

### Example 3:

### Obtainment of the biomass of Galleria mellonella larvae with anti-inflammatory activity

Pupae of *Galleria mellonella* are taken out the refrigerator, where were stored at the temperature of 3-5 °C and are left at the indoor temperature for 2 hours. With the passage of this time, the pupae are transferred to the closed containers containing a small amount of the nutrient medium (1/10 of the volume of the container) for their subsequent development and transition to the phase of imago and oviposition. For the initiation of the hatching of larvae from eggs in the dark containers, it is maintained the temperature of 27 °C and a relative humidity of 75%. The first instar larvae appear 48 hours after the pupae are placed in the containers. The process is monitored, over a period of time during which 80% yield is provided, but not more than 6 hours.

The used nutrient medium has the following composition, g/kg:

| | |
|---|---|
| Wheat flour | 190 g |
| Corn flour | 180 g |
| Wheat bran | 125 g |
| Yeast extract | 18 g |
| Powdered milk | 130 g |
| Bee honey | 90 g |
| Glycerine, | 90 g |
| Wax powder | 55 g |
| Pinobanksin | 0,03 g |
| Pinocembrin | 0,03 g |
| Purified water up to 1000 g; | |

New containers with nutrient medium are prepared. 6 hours after the start of the hatching, the larvae of the first generation are selected by the tweezers from the containers in which they appeared and are placed in the prepared containers.

Growth of larvae lasts 21 days since the date of the first transfer. The temperature of the container is maintained at the level of 27 °C and the relative humidity constitutes 75% in the dark conditions.

During the given period, on the 7^{th} day and the 14^{th} day, two transfers of larvae to a fresh nutrient medium are carried out.

For the obtainment of the biomass of larvae with a high anti-inflammatory activity, the collection of larvae occurs on the 21^{st} day of cultivation. Larvae are washed twice by four times volumes of physiological solution and twice more by four times volumes of purified water.

In the obtained biomass are determined the quantitative and qualitative indicators. The obtained results are demonstrated in the table 3.

**Table 3**

| **Parameter** | **According to the closest solution** | **According to the proposed method** |
|---|---|---|
| Cost of one kg of nutrient medium | 100% | 25% |
| Amount of biomass of larvae (mg) obtained from one g of medium | 121,3±2,7 | 160,0±0,8 |
| Decrease of the amount of the released IL₈ (% of control) | 28,5±0,7 | 37,1±0,2 |

The data in the Table 3 demonstrate that the amount of the biomass obtained using one gram of medium increases by 31,9% compared to the prototype. Also, a higher level of anti-inflammatory activity was observed, manifested in the decrease of the level of the pro-inflammatory factor - interleukin 8 (decrease in the proposed method by 37,1% compared with the decrease of 28,5% for the prototype).

### Sources:

1. 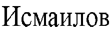 B. 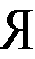 ., 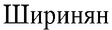 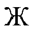 .A., 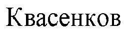 O. 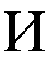 . 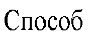 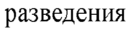 Galleria mellonella, 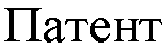 RU 2210210, publicat la 20.08.2003.
2. Adeline L. Jorj 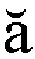 o, Luciane D. Oliveira, Liliana Scorzoni, Livia Mara A. Figueiredo-Godoi, Marcia Cristina A. Prata, Antonio Olavo C. Jorge & Juliana C. Junqueira (2018) From moths to caterpillars: Ideal conditions for Galleriamellonella rearing for in vivo microbiological studies, Virulence, 9:1, 383-389.
3. C.van Zyl, A.P.Malan. Cost-Effective Culturing of Galleria mellonella and Tenebrio Molitor and Entomopathogenic Nematode Production in Various Hosts. African Entomology 23(2):361 -375. 2015.
4. Ciuhrii V. Complex de origine enlomologic 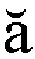 in propilenglicol cu ac 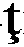 iune cheralolilic 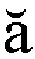 , procedeu de obtinere a acestuia, produse farmaceutice 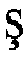 i cosmetice pe baza lui (variante). Brevet de inven 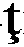 tie, MD 4198, BOPI nr.2, 2013.

## Claims

1. Method of obtainment of the biomass of Galleria Mellonella larvae with high biological activity which consists in placing the pupae in containers with nutrient medium, maintaining them at a temperature of 27°C
and a humidity of 75% until the hatching of the first instar larvae, transferring the larvae after 6 hours after hatching in other containers with nutrient medium, their cultivation at a temperature of 27°C
and humidity of 75%, the nutrient cultivation medium having the following composition, g/kg:
| | |
|---|---|
| Wheat flour | 185 - 200 |
| Corn flour | 180 - 190 |
| Wheat bran | 110 - 130 |
| Yeast extract | 15 - 20 |
| Milk powder | 120 - 140 |
| Bee honey | 75 - 95 |
| Glycerol | 80 - 90 |
| Wax powder | 40 - 55 |
| Pinobanksin | 0.02 - 0.03 |
| Pinocembrin | 0.02 - 0.03 |
| Purified water | the rest |
**characterized in that** the cultivation of larvae takes place optimally for 19-21 days, respectively: 19 days - for biomass with high keratolytic activity; 20 days - for biomass with high antioxidant activity and 21 days - for biomass with high anti-inflammatory activity, with the transfer of larvae at least 2 times to fresh nutrient medium and the collection of larvae.

## Patentansprüche

1. Verfahren zur Gewinnung der Biomasse von Galleria mellonella-Larven mit hoher biologischer Aktivität, das darin besteht, die Puppen in Behältern mit Nährmedium unterzubringen, sie bei einer Temperatur von 27°C und einer Feuchtigkeit von 75% bis zum Schlüpfen der Larven des ersten Larvenstadiums zu halten, die Larven nach 6 Stunden nach dem Schlüpfen in andere Behälter mit Nährmedium zu überführen, sie bei einer Temperatur von 27°C und einer Feuchtigkeit von 75% zu züchten, wobei das nährende Aufzuchtmedium die folgende Zusammensetzung aufweist, g/kg:
| | |
|---|---|
| Weizenmehl | 185 - 200 |
| Maisstärke | 180 - 190 |
| Weizenkleie | 110 - 130 |
| Hefeextrakt | 15 - 20 |
| Milchpulver | 120 - 140 |
| Bienenhonig | 75 - 95 |
| Glycerin | 80 - 90 |
| Wachspulver | 40 - 55 |
| Pinobanksin | 0,02 - 0,03 |
| Pinocembrin | 0,02 - 0,03 |
| aufbereitetes Wasser | der Rest |
**dadurch gekennzeichnet, dass** die Aufzucht der Larven optimal über 19-21 Tage erfolgt: 19 Tage - für Biomasse mit hoher keratolytischer Aktivität; 20 Tage - für Biomasse mit hoher antioxidativer Aktivität und 21 Tage - für Biomasse mit hoher entzündungshemmender Aktivität, wobei die Larven mindestens zweimal auf ein frisches Nährmedium überführt werden und die Larven gesammelt werden.

## Revendications

1. Procédé d'obtention de la biomasse de larves de Galleria Mellonella à haute activité biologique, qui consiste à placer les pupes dans des récipients avec un milieu nutritif, à les maintenir à une température de Z r 270C Z et une humidité de 75% jusqu'à l'éclosion des larves du premier stade, à transférer les larves après 6 heures après l'éclosion dans d'autres récipients avec un milieu nutritif, à les cultiver à une température de Z r 270C Z et une humidité de 75%, le milieu de culture nutritif ayant la composition suivante, g/kg :
| | |
|---|---|
| Farine de blé | 185 - 200 |
| Farine de maïs | 180 - 190 |
| Son de blé | 110 - 130 |
| Extrait de levure | 15 - 20 |
| Lait en poudre | 120 - 140 |
| Miel d'abeille | 75 - 95 |
| Glycérol | 80 - 90 |
| Poudre de cire | 40 - 55 |
| Pinobanksine/ Pinobanquesin | 0.02 - 0.03 |
| Pinocembrine | 0.02 - 0.03 |
| Eau purifiée | le reste |
**caractérisé en ce que** la culture des larves se déroule de manière optimale pendant 19 à 21 jours, respectivement : 19 jours - pour la biomasse à haute activité kératolytique ; 20 jours - pour la biomasse à haute activité antioxydante et 21 jours - pour la biomasse à haute activité anti-inflammatoire, avec le transfert des larves au moins 2 fois dans un milieu nutritif frais et la collecte des larves.
